Europäisches Patentamt

European Patent Office

Office européen des brevets

(19)

(11) Veröffentlichungsnummer: **0 306 797**

**A2**

(12) **EUROPÄISCHE PATENTANMELDUNG**

(21) Anmeldenummer: 88114089.1

(22) Anmeldetag: 30.08.88

(51) Int. Cl.4: **C08F 220/58** , //G01N33/543, **C12N11/08**

Patentansprüche für folgenden Vertragsstaat: ES.

(30) Priorität: 11.09.87 DE 3730515

(43) Veröffentlichungstag der Anmeldung:
**15.03.89 Patentblatt 89/11**

(84) Benannte Vertragsstaaten:
**AT BE CH DE ES FR GB IT LI LU NL SE**

(71) Anmelder: **BEHRINGWERKE
Aktiengesellschaft
Postfach 1140
D-3550 Marburg 1(DE)**

(72) Erfinder: **Kapmeyer, Wolfgang, Dr.
Reinhardswaldstrasse 5
D-3550 Marburg(DE)**
Erfinder: **Dengler, Michael, Dr.
Unter den Eichen 23
D-3550 Marburg(DE)**

(74) Vertreter: **Meyer-Dulheuer, Karl-Hermann, Dr.
et al
HOECHST Aktiengesellschaft Zentrale
Patentabteilung Postfach 80 03 20
D-6230 Frankfurt/Main 80(DE)**

(54) **Dispersionspolymere, Verfahren zu ihrer Herstellung und ihre Verwendung.**

(57) Es werden Dispersionspolymere aus einer Verbindung oder mehreren Verbindungen der Formel I

$$CH_2 = CR_1\text{-}CO\text{-}NH\text{-}(CH_2)_n\text{-}CH(OR_2)OR_3 \qquad I$$

worin
n = 1 - 6 und
$R_1$ = H oder $CH_3$ sind und
$R_2$ und $R_3$ gleich oder unterschiedlich sind und
$(CH_2)_m\text{-}CH_3$ mit m 0 - 7 oder
$-C(X)$ (Y)Z mit X, Y und Z = $(CH_2)_p CH_3$ und
p = 1 - 3, wobei X, Y, und Z gleich oder unterschiedlich sein können, bedeuten, und gegebenenfalls einem weiteren Vinylderivat oder weiteren Vinylderivaten in einem wässrigen Medium,
ein Verfahren zu ihrer Herstellung sowie ihre Verwendung zur Herstellung von Konjugaten mit biologisch aktiven Substanzen beschrieben.

**EP 0 306 797 A2**

## Dispersionspolymere, Verfahren zu ihrer Herstellung und ihre Verwendung

Die Erfindung betrifft Dispersionspolymere, die aus Latexpartikeln bestehen, die ein Polymerisat von Vinylmonomeren sind, die eine Acetalgruppe tragen oder ein Copolymerisat solcher Monomeren mit anderen Vinylmonomeren tragen, sowie ein Verfahren zu ihrer Herstellung. Diese Polymere können zur Herstellung von biologisch aktiven Latexkonjugaten, die für serologische oder immunologische Bestimmungsverfahren geeignet sind, verwendet werden, indem biologisch aktive Substanzen, die über freie Aminogruppen verfügen, an sie gebunden werden.

Es ist bekannt, die Empfindlichkeit serologischer oder immunologischer Bestimmungsverfahren durch die Verwendung von Indikator- oder Trägerteilchen zu erhöhen, die mit dem entsprechenden immunologischen Reagenz beladen sind. Als Trägerteilchen können Latexpartikel mit einem Durchmesser von 0,02 bis 5μm verwendet werden.

Aus der Europäischen Patentanmeldung EP-A-0 080 614 (USP 4,448,908) sind Latexpartikel bekannt, die über Säureamidgruppen gebundene Acetal-Funktionen enthalten. In einem wässrigen Medium vorgefertigte Latexkerne werden mit Vinylmonomeren, die über Säureamidgruppen gebundene Acetalfunktionen enthalten, angequollen. Diese Vinylmonomere, die ausreichend wasserunlöslich sein müssen, werden dann zusammen mit weiteren Monomeren, die hydrophiler oder ionischer Natur sein können, copolymerisiert. Derartige Reagenzien lassen sich zur nephelometrischen Bestimmungen von Serumproteinen, beispielsweise von C-reaktivem Protein, einsetzen.

Das in der EP-A-0 080 614 beschriebene Verfahren ist aufwendig, da die Herstellung der Latexpartikel in zwei Stufen erfolgt, wobei zunächst Kerne hergestellt werden. Außerdem gelingt das Anquellen der Kerne für die Schalenpolymerisation nicht mit allen Vinylmonomeren, sondern nur mit solchen, die ausreichend wasserunlöslich sind.

Weiterhin führt das bisherige Kern-Schale-Polymerisationsverfahren nur zu Latexpartikeln bestimmter Größen, ausgehend von vorhandenen Latexkernen.

Daher bestand ein Bedarf an einem einstufigen Copolymerisationsverfahren zur Herstellung von Dispersionspolymeren beliebiger Größe unter Verwendung von Vinylverbindungen, die Acetalgruppen tragen.

Es wurde nun überraschend gefunden, daß die geschilderten Nachteile des Standes der Technik überwunden werden können, wenn man Trägerpartikel verwendet, die dadurch hergestellt sind, daß in einem wäßrigen Medium Acryl-oder Methacrylmonomeren, die über Säureamidgruppen gebundene Acetal-Funktionen enthalten, zusammen mit weiteren Vinylmonomeren polymerisiert werden.

Gegenstand der Erfindung ist somit ein Dispersionspolymer aus einer Verbindung oder mehreren Verbindungen der Formel I

$$CH_2 = CR_1\text{-}CO\text{-}NH\text{-}(CH_2)_n\text{-}CH(OR_2)OR_3 \qquad I$$

worin
n = 1 - 6 und $R_1$ = H oder $CH_3$ sind und
$R_2$ und $R_3$ gleich oder unterschiedlich sind und
$\text{-}(CH_2)_m\text{-}CH_3$ mit m 0 - 7 oder $\text{-}C(X)(Y)Z$ mit X, Y und Z = $(CH_2)_pCH_3$ und
p = 1 - 3, wobei X, Y, und Z gleich oder unterschiedlich sein können,
bedeuten, und gegebenenfalls einem weiteren Vinylderivat oder weiteren Vinylderivaten in einem wässrigen Medium.

In Abbildung 1 sind die Ergebnisse von CRP-Bestimmungen mit einem erfindungsgemäßen Polymer in einem Turbidimetrie-Test (Abszisse) im Vergleich mit dem klassischen nephelometrischen Test am BNA-System (NA-Latex-CRP-Test, Behringwerke AG) auf der Ordinate aufgetragen.

In Abbildung 2 sieht man die Ergebnisse von IgE-Messungen mit einem erfindungsgemäßen Polymer in einem Turbidimetrie-Test (Abszisse) und dem klassischen Enzymimmunoassay (®Enzygnost IgE, Behringwerke AG) auf der Ordinate.

In Abbildung 3 sind die Ergebnisse von AFP-Messungen mit einem erfindungsgemäßen Polymer in einem Turbidimetrie-Test (Abszisse) und dem klasssischen Enzymimmunoassay (®Enzygnost IgE, Behringwerke AG) auf der Ordinate dargestellt.

Bevorzugt verwendet man als Verbindung der Formel I Acryl- oder Methacrylamidoalkanal- dialkylacetal mit $C_2$- bis $C_8$- Alkyl im Acetalteil. Besonders geeignet sind Acryl- oder Methacrylamidoacetaldehyd-di-n-pentylacetal.

Geeignete Vinylpolymere, die nicht unter die Formel I fallen, sind beispielsweise Styrol, Vinylnaphthalin, Vinyltoluol, Methacrylsäure, Acrylsäure oder Crotonsäure.

Vorteilhaft ist auch die zusätzliche Verwendung von wasserlöslichen Monomeren aus der Gruppe der hydroxysubstituierten Acryl- oder Methacrylsäureester bzw. Amide, wie z.B. N-(2,3-Dihydroxypropyl)methacrylamid, N-(2-Hydroxypropl)methacrylamid oder 2-Hydroxypropylmethacrylat. Auch die zusätzliche Verwendung von wasserlöslichen Monomeren mit Betainstruktur, wie z.B. N-(3-Sulfopropyl)-N-methacryloxyethyl-N,N-dimethylammoniumbetain oder N-(3-Sulfopropyl)-N-methacrylamidopropyl-N,N-dimethylammoniumbetain kann vorteilhaft sein.

Die erfindungsgemäßen Dispersionen ("Latices") können durch Polymerisation oder gegebenenfalls Copolymerisation von einem oder mehreren Vinylmonomeren nach Formel I und gegebenenfalls weiteren Vinylmonomeren in Gegenwart von ionischen und/oder nicht ionischen Detergenzien hergestellt werden.

Gegenstand der Erfindung ist deshalb auch ein Verfahren zur Herstellung eines Polymeren wie oben definiert, dadurch gekennzeichnet, daß eine Verbindung oder mehrere Verbindungen der Formel I und gegebenenfalls ein weiteres Vinylderivat oder weitere Vinylderivate in einem wässrigen Medium in Gegenwart eines Emulgators und eines radikalbildenden Initiatorspolymerisiert wird.

In Frage kommende Emulgatoren (Detergenzien) sind beispielsweise Polyglykolether mit langkettigen, aliphatischen Alkoholen, die vorzugsweise 10-20 Kohlenstoffatome aufweisen, oder Alkylphenole, deren Alkylrest vorzugsweise 6-12 Kohlenstoffatome enthält, oder Dialkylphenolen oder Trialkylphenolen, deren Alkylreste vorzugsweise verzweigte Alkylreste mit jeweils 3-12 Kohlenstoffatomen darstellen. Beispiele hierfür sind Umsetzungsprodukte von Äthylenoxyd mit Laurylalkohol, Stearylalkohol, Oleylalkohol, Kokosfettalkohol, Octylphenol, Nonylphenol, Diisopropylphenol, Triisopropylphenol, Di-t-butylphenol und Tri-t-butylphenol. Reaktionsprodukte des Ethylenoxids mit Polypropylenglykol oder Polybutylenglykol sind ebenfalls geeignet.

Von den ionischen Emulgatoren sind vor allem anionische Emulgatoren geeignet, insbesondere Alkali- oder Ammoniumsalze von Alkylsulfonaten, Arylsulfonaten oder Alkylarylsulfonaten sowie von den entsprechenden Sulfaten, Phosphaten oder Phosphonaten, die gegebenenfalls Oxyethylen-Einheiten zwischen dem jeweiligen Kohlenwasserstoffrest und der anionischen Gruppe aufweisen. Beispiele hierfür sind Natriumdodecylsulfat, Natriumlaurylsulfat, Natriumoctylphenolglykolethersulfat, Natriumdodecylbenzolsulfonat, Natriumlauryldiglykolsulfat, Ammonium-tri-t-butylphenolpentaglykolsulfat und Ammonium-tri-t-butylphenoloctaglykolsulfat. Bevorzugt eingesetzt wird eine Mischung von Natriumdodecylsulfat und Octylphenoxypolyethoxyethanol (®Triton X 405).

Vorteilhafterweise setzt man der aus den Monomeren bestehenden Mischung bis zu 20 Gew.%, bezogen auf die Monomerenmischung, Dimethylformamid oder andere Substanzen, die die Viskosität herabsetzen, zu.

Die Polymerisation oder Copolymerisation kann nach an sich üblichen Verfahren durchgeführt werden. Bevorzugte Ausführungsform des erfindungsgemäßen Verfahrens ist jedoch das Dosierverfahren, bei dem das Monomer oder die Monomerenmischung unter ständigem Rühren der wäßrigen Lösung von Emulgator und dem radikalbildenden Initiator bei Polymerisationsbedingungen, d.h. bei einer Temperatur von +10°C bis +120°C, vorzugsweise +50°C bis +90°C, zugetropft wird.

Anschließend wird das Polymerisat von überschüssigen Monomeren, Resten von Initiator und Emulgator nach bekannten Verfahren befreit. Vorteilhafterweise unterwirft man das Polymerisat einer Dialyse, z.B. gegen $NaHCO_3$-Puffer (0,01 bis 0,05 Gew.%).

Gegenstand der Erfindung ist weiterhin die Verwendung eines erfindungsgemäßen Polymeren oder Copolymeren zur Herstellung eines Konjugats mit einer biologisch aktiven Substanz.

Zur Herstellung einer solchen Konjugat-Dispersion, nachfolgend auch als Latexkonjugat bezeichnet, kann eine Suspension der oben beschriebenen saatpolymerisierten Latexpartikel auf einen pH-Wert unter 5, vorzugsweise unter 3, eingestellt und mit dem zu bindenden immunologisch aktiven Material, wie z.B. einem Antikörper oder Antigen, inkubiert werden. Die labilen Bindungen zwischen einer Aminogruppe des Proteins und dem freigesetzten Aldehyd auf dem erfindungsgemäßen Latexpartikel werden nach bekannten Verfahren reduziert. Bevorzugt wird eine Lösung von Natriumcyanborhydrid in neutralem Puffer hierfür verwendet. Man trennt eventuell ungebundenes immunologisch aktives Material oder andere Verunreinigungen aus dem Reaktionsansatz ab. Dies geschieht zweckmäßigerweise durch Zentrifugieren oder Waschen auf geeigneten Membranen.

Die erfindungsgemäßen, saatpolymerisierten Latices zeichnen sich durch gute Stabilität aus. Sie eignen sich zur Herstellung besonders empfindlicher Reagenzien. Die Nachteile herkömmlicher Reagenzien kommen nämlich auch dadurch zum Ausdruck, daß die mit ihnen durchgeführten nephelometrischen oder turbidimetrischen Messungen zu Ergebnissen führen, die mit denen eines Enzymimmunoassays nicht gut übereinstimmen. Nach Beispiele 1 und 2 wurde ein Reagenz zur Bestimmung von C-reaktivem Protein (CRP) hergestellt. Es wurden turbidimetrische Messungen nach Beispiel 3 mit diesem Reagenz durchgeführt.

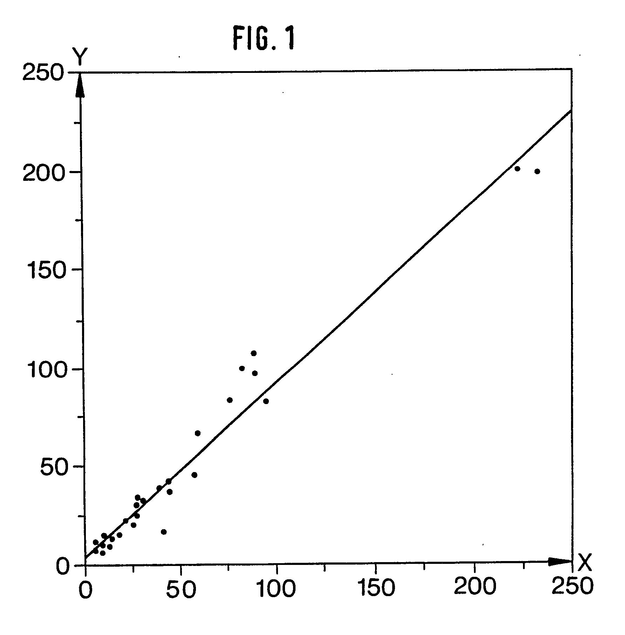

Das CRP-Reagenz erlaubt Messungen zwischen ca. 5 und 250 mg/l CRP. Der diagnostisch wichtige Meßbereich wird also voll erfaßt. Wie aus Abbildung 1 ersichtlich, wird eine gute Übereinstimmung mit den Ergebnissen des nephelometrischen Testes (Na-Latex-CRP-Reagenz, Behringwerke AG) erzielt. Die Korrelationskonstanten lauten: $\gamma$ - (Turbidimetrie) = 0,909 X (BNA) + 5,821 mg/l, r = 0,967. Mit den erfindungsgemäßen Polymeren lassen sich auch Reagenzien für Spurenproteinmessungen herstellen.

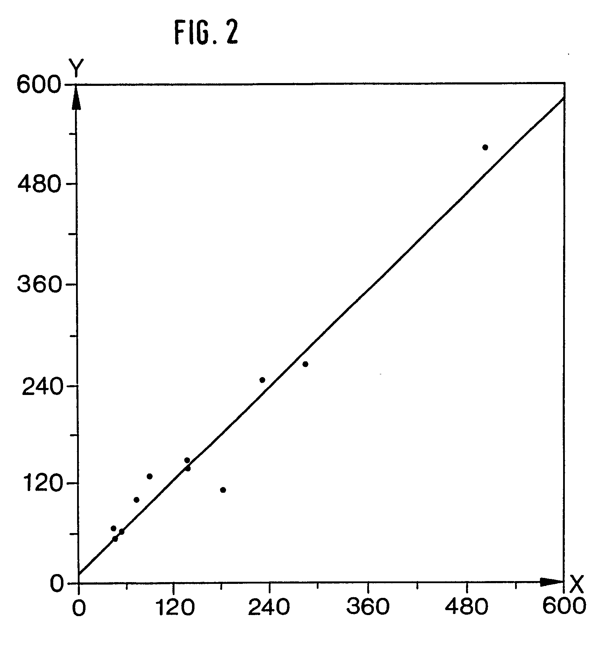

Nach Beispiel 4 wurde ein Reagenz zur turbidimetrischen Bestimmung von Immunglobulin E (IgE) und nach Beispiel 6 ein Reagenz zur turbidimetrischen Bestimmung von alpha-Fetoprotein (AFP) hergestellt. Die turbidimetrische Messung von IgE erfolgte nach Beispiel 5 und die turbidimetrische Messung von AFP nach Beispiel 7. Der Meßbereich für das IgE-Reagenz liegt zwischen 30 und 2000 IU/ml. Für Serumkollektive besteht eine gute Übereinstimmung mit den Ergebnissen des Enzymimmunoassays (Enzygnost IgE, Behringwerke AG), wie Abbildung 2 zeigt. Die Korrelationskonstanten lauten: $\gamma$ (Turbidimetrie) = 0,962 X (EIA) + 11,69 IU/ml, r = 0,973.

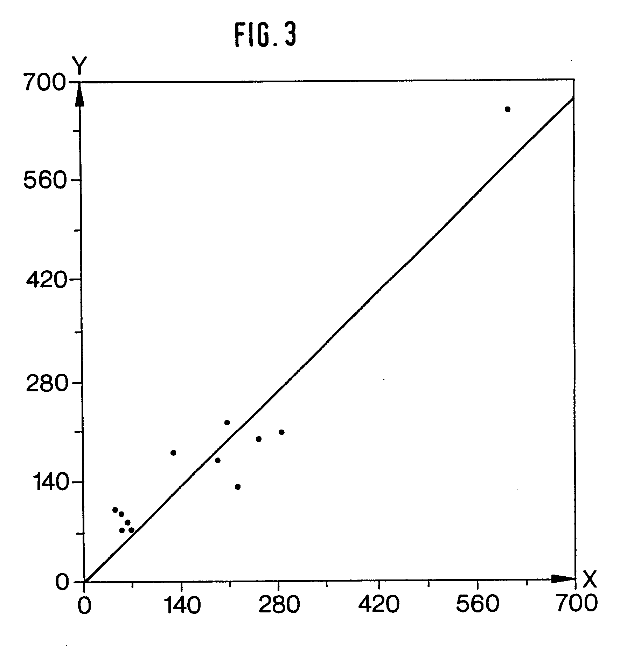

Der Meßbereich für das AFP-Reagenz liegt zwischen 50 und 1500 ng/ml. Für Serum- bzw. Plasmaproben wurde eine befriedigende Übereinstimmung mit den Ergebnissen des Enzymimmunoassays (Enzygnost AFP, Behringwerke AG) gefunden, wie Abbildung 3 zeigt. Die Korrelationskonstanten lauten: $\gamma$ (Turbidimetrie) = 0,965 X (EIA) + 4,31 ng/ml, r = 0,945.

Die Latexkonjugate können in allen diagnostischen Verfahren eingesetzt werden, die Teilchengrößenveränderungen messen, beispielsweise in qualitativen und semiquantitativen Bestimmungen von Substanzen mit Hilfe von visuellen Latexagglutinationstesten sowie in nephelometrischen oder turbidimetrischen Bestimmungen von Spurenproteinen im direkten oder kompetitiven Agglutinationstest oder im Latex-Hapten-Inhibitionstest.

Die folgenden Beispiele erläutern die Erfindung.

Beispiel 1

Saatpolymerisat

77 ml destilliertes Wasser ($N_2$ gesättigt) wurden zusammen mit 2 mg Octylphenoxypolyethoxyethanol (®Triton X 405, gelöst in 2 ml destilliertem Wasser) sowie 50 mg Natriumdodecylsulfat in das Polymerisationsgefäß gegeben und der Sauerstoff entfernt. Weiterhin wurden 1 ml einer Kaliumperoxydisulfatlösung, 16 mg/ml in destilliertem Wasser, hinzugegeben. Man stellte eine Mischung aus 0,4 ml Styrol, 0,4 ml Methacrylamidoacetaldehyddi-n-pentylacetal und 0,002 ml Methacrylsäure her.

Die Mischung der Monomeren wurde zur kräftig gerührten wäßrigen Lösung bei +70° C während 60 Minuten langsam zugetropft. Anschließend wurde weitere 5 Stunden bei gleicher Temperatur weitergerührt. Nach Abkühlung auf Raumtemperatur und Filtration durch ein Faltenfilter erhielt man 78 ml des Polymerisates. Man dialysierte anschließend ca. 20 Stunden gegen Na $HCO_3$-Puffer (0,25 g/l, pH 8-8,2). Man erhielt 80 ml einer Latex-Suspension mit einem Feststoffgehalt von 0,71 Gew.%.

Beispiel 2

Bindung von Anti-CRP-Antikörpern an ein erfindungsgemäßes Polymer

Ein Antiserum wurde durch Immunisierung von Kaninchen mit gereinigtem C-reaktiven Protein (CRP) gewonnen. Daraus wurde nach einem Ionenaustauschverfahren unter Verwendung von DE-32 Zellulose die Fraktion der gamma-Globuline gewonnen. Diese wurde mit einem Proteingehalt von ca. 10 mg/ml eingesetzt.

3,22 ml des im Beispiel 1 beschriebenen Polymerisates wurden mit 0,1 ml Anti-CRP-Antikörper-Lösung gemischt. Dann wurden 0,05 ml einer 20 g/100 ml wässrigen Lösung von Eikosaoxyethylen-sorbitan-laurylsäureester (®Tween 20) hinzugegeben und das Ganze nochmals gemischt. Hierzu gaben wir 0,05 ml 1 N HCl, so daß ein pH-Wert von ca. 2 erreicht wurde.

Nach einer Inkubationszeit von 30 Minuten bei Raumtemperatur setzten wir 0,25 ml gesättigte wäßrige Dinatriumhydrogenphosphat-Lösung (pH 6,5) und 0,25 ml Natriumcyanborhydrid (25 mg/ml) hinzu und mischten gut durch.

Anschließend erfolgte eine Inkubation von einer Stunde bei Raumtemperatur. Dieser Beladungsansatz wurde sodann 30 Minuten mit ca. 50000 g zentrifugiert (Beckman Zentrifuge 20000 rpm). Der Überstand wurde verworfen. Der Rückstand wurde in 2 ml Glycin-NaCl-Puffer (0,1M Glycin, 0,17 M NaCl, 0,5 g/100 ml Eikosaoxyethylen-sorbitan-laurylsäureester (®Tween 20) pH 8,2) resuspendiert.

Anschließend erfolgte eine Ultraschall-Behandlung (Bronson Sonyfier B 15) für 2 Sekunden. Das so redispergierte Reagenz wurde im Volumenverhältnis 1:40 mit 0,025 M Imidazol-Puffer pH 8,2 (3g/100 ml Saccharose, 0,05 g/100 ml Human Albumin, 0,05 g/100 ml $NaN_3$) verdünnt.

Beispiel 3

Turbidimetrische Messung von CRP-Konzentration in Serumproben

Das nach Beispiel 2 durch Bindung von Anti-CRP-Antikörpern an erfindungsgemäße Polymere hergestellte CRP-Reagenz wurde zur Messung von CRP in Patientenseren eingesetzt.

Als Standard wurde ein CRP-Standardserum mit einer CRP-Konzentration von 26 mg/l (Behringwerke AG) verwendet. Der Standard wurde mit physiologischer Kochsalz-Lösung 1:10, 1:20, 1:40, 1:80, 1:160 und 1:320 verdünnt. Die Patientensera wurden 1:100 mit physiologischer Kochsalz-Lösung verdünnt.

Zur Messung wurden 80 $\mu$l Patientenserum-Verdünnung bzw. Standardserum-Verdünnung mit 450 $\mu$l eines Reaktionspuffers (0,1 M Glycin, 0,17 M NaCl, 4 g/100 ml Polyethylenglykol 6000, 0,5 g/100 ml ®Tween 20, pH 8,2) und 150 $\mu$l CRP-Reagenz nach Beispiel 2 in Mikro-Küvetten (Fa. Sarstedt 1 x 0,4 cm) gemischt. Für die Küvetten wurde dann die Extinktion im Eppendorf-Photometer bei einer Wellenlänge von 334 nm nach 10 Sekunden und nach 3 Minuten gemessen. Die Meßwerte wurden als Differenz zwischen den 10 Sekunden- und 3 Minutenwerten berechnet.

Die Referenzkurve für die Messung des Standards wurde auf halblogarithmischem Papier mit Konzentrationen auf der Abszisse und den Extinktions-werten auf der Ordinate gezeichnet und daran wurden die Meßwerte für die Patientenseren ausgewertet.

## Beispiel 4

Bindung von Anti-IgE-Antikörpern an ein erfindungsgemäßes Polymerisat nach Beispiel 1

Ein Antiserum wurde durch Immunisierung von Kaninchen mit gereinigtem Immunglobulin E (IgE) gewonnen. Daraus wurde nach einem Immunadsorptionsverfahren unter Verwendung von Trägergebundenem IgE der Antikörper gewonnen. Es wurde anschließend auf einen Proteingehalt von ca. 10 mg/ml konzentriert.

5,64 ml des nach Beispiel 1 hergestellten Polymerisates wurden mit 0,1 ml Anti-IgE-Antikörper-Lösung gemischt.

Dann wurden 0,05 ml einer 20 g/100 ml enthaltenden wässrigen Lösung von Eikosaoxyethylen-sorbitan-laurylsäureester (®Tween 20) hinzugegeben und das Ganze nochmals gemischt. Hierzu wurden 0,18 ml 1 N HCl gegeben, so daß ein pH-Wert von ca. 2 erreicht wurde. Nach einer Inkubationszeit von 30 Minuten bei Raumtemperatur wurden 0,25 ml einer gesättigten wäßrigen Dinatrium hydrogenphosphat-Lösung (pH 6,5) und 0,25 ml Natriumcyanborhydrid-Lösung (25 mg/ml) hinzugeben und gut durchmischt. Anschließend erfolgte eine Inkubation von einer Stunde bei Raumtemperatur. Dieser Beladungsansatz wurde sodann 30 Minuten mit ca. 50000 g zentrifugiert (Beckman Zentrifuge 20000 rpm). Der Überstand wurde verworfen. Der Rückstand wurde in 2 ml Glycin-NaCl-Puffer (0,1 M Glycin, 0,17 M NaCl, 0,5 g/100 ml Eikosaoxyethylen-sorbitan-laurylsäureester pH 8,2) resuspendiert. Anschließend erfolgte eine Ultraschall-Behandlung (Bronson-Sonyfier B15) für 2 Sekunden. Das so redispergierte Reagenz wurde im Volumenverhältnis 1:20 mit 0,025 M Imidazol-Puffer pH 8,2 (3 g/100 ml Saccharose, 0,05 g/100 ml Human Albumin, 0,05 g/100 ml $NaN_3$) verdünnt.

## Beispiel 5

Turbidimetrische Messung von IgE-Konzentrationen in Serumproben

Das nach Beispiel 4 durch Bindung von Anti-IgE-Antikörpern an erfindungsgemäße Latexpräparationen hergestellte Reagenz zur Bestimmung von IgE wurde zur Messung von IgE in Patientenseren eingesetzt.

Als Standard wurde ein IgE-Standardserum mit einer IgE-Konzentration von 433 IU/ml (Fa. Behringwerke AG) verwendet. Der Standard wurde mit physiologischer Kochsalz-Lösung zusätzlich 1:2, 1:4, 1:8, 1:16, 1:32 und 1:64 verdünnt. Die Patientensera wurden 1:5 mit physiologischer Kochsalz-Lösung verdünnt.

Zur Messung wurden 80 $\mu$l Patientenserum-Verdünnung bzw. Standardserum-Verdünnung mit 450 $\mu$l eines Reaktionspuffers (0,1 M Glycin, 0,17 M NaCl, 4 g/100 ml Polyethylenglykol 6000, 0,5 g/100 ml ®Tween 20, pH 8,2) und 40 $\mu$l einer Lösung von 1:32 verdünntem Kaninchenserum in 6 g/100 ml enthaltender NaCl-Lösung mit 4 g/100 ml ®Tween 20, sowie 150 $\mu$l IgE-Reagenz nach Beispiel 4 in Mikro-Küvetten (Fa. Sarstedt 1 x 0,4 cm) gemischt. Für die Küvetten wurde dann Extinktionswerte im Eppendorf-Photometer bei einer Wellenlänge von 334 nm nach 10 Sekunden und nach 3 Minuten gemessen. Die Meßwerte wurden als Differenz zwischen den 10 Sekunden- und 3 Minutenwerten berechnet.

Die Referenzkurve für die Messung des Standards wurde auf halblogarithmischem Papier mit IgE-Konzentrationen auf der Abszisse und den Extinktionswerten auf der Ordinate gezeichnet und daran wurden die Meßwerte für die Patientenseren ausgewertet.

## Beispiel 6

Bindung von Anti-AFP-Antikörpern an ein erfindungsgemäßes Polymer

Ein Antiserum wurde durch Immunisierung von Kaninchen mit gereinigtem alpha-Fetoprotein (AFP) gewonnen. Daraus wurde nach einem Immunadsorptionsverfahren unter Verwendung von Trägergebundenem AFP der Antikörper gewonnen.

Es wurde anschließend auf einen Proteingehalt von ca. 10 mg/ml konzentriert.

5,64 ml des nach Beispiel 1 hergestellten Polymerisates wurden mit 0,1 ml Anti-AFP-Antikörper-Lösung gemischt.

Dann wurden 0,05 ml einer 20 g/100 ml enthaltenden wässrigen Lösung von Eikosaoxyethylen-sorbitan-laurylsäureester (®Tween 20) hinzugegeben und das Ganze nochmals gemischt. Hierzu wurden 0,18 ml 1 N HCl gegeben, so daß ein pH-Wert von ca. 2 erreicht wurde. Nach einer Inkubationszeit von 30 Minuten bei Raumtemperatur wurden 0,25 ml einer gesättigten wäßrigenDinatriumhydrogenphosphat-Lösung (pH 6,5) hinzugesetzt und 0,25 ml Natriumcyanborhydrid-Lösung (25 mg/ml) hinzugeben und gut durchmischt. Anschließend erfolgte eine Inkubation von einer Stunde bei Raumtemperatur.

Dieser Beladungsansatz wurde sodann 30 Minuten mit ca. 50000 g zentrifugiert (Beckman Zentrifuge 20000 rpm). Der Überstand wurde verworfen. Der Rückstand wurde in 2 ml Glycin-NaCl-Puffer (0,1 M Glycin, 0,17 M NaCl, 0,5 g/100 ml ®Tween 20, pH 8,2) resuspendiert.

Anschließend erfolgte eine Ultraschall-Behandlung (Bronson Sonyfier B 15) für 2 Sekunden. Das so redispergierte Reagenz wurde im Volumenverhältnis 1:40 mit 0,025 M Imidazol-Puffer pH 8,2 (3 g/100 ml Saccharose, 0,05 g/100 ml Human Albumin, 0,05 g/100 ml NaN$_3$) verdünnt.

Beispiel 7

Turbidimetrische Messung von AFP-Konzentrationen in Serumproben

Das nach Beispiel 6 durch Bindung von Anti-AFP-Antikör pern an erfindungsgemäße Latexpräparationen (nach Beispiel 1) hergestellte Reagenz zur Bestimmung von AFP wurde zur Messung von AFP in Patientenseren eingesetzt.

Als Standard wurde ein AFP-Standardserum mit einer AFP-Konzentration von 300.000 ng/ml (Fa. Behringwerke AG) verwendet.

Der Standard wurde in einem AFP-freien Serumpool stufenweise auf 600 ng/ml verdünnt. Von dieser Verdünnung wurde eine Standardreihe mit abnehmenden AFP-Konzentrationen in den Verdünnungen 1:2, 1:4, 1:8, 1:16, 1:32 und 1:64 mit physiologischer Kochsalz-Lösung hergestellt. Die Patientensera wurden 1:5 mit physiologischer Kochsalz-Lösung verdünnt.

Zur Messung wurden 80 µl Patientenserum-Verdünnung bzw. Standardserum-Verdünnung mit 450 µl eines Reaktionspuffers (0,1 M Glycin, 0,17 M NaCl, 4 g/100 ml Polyethylenglykol 6000, 0,5 g/100 ml ®Tween 20, pH 8,2) und 10 µl einer Lösung von 1:8 verdünntem Kaninchenserum in einer Lösung von 8 g/100 ml NaCl und 5 g/100 ml ®Tween 20 sowie mit 150 µl AFP-Reagenz nach Beispiel 6 in Mikro-Küvetten (Fa. Sarstedt 1 x 0,4 cm) gemischt. Für die Küvetten wurde dann Extinktionswerte im Eppendorf-Photometer bei einer Wellenlänge von 334 nm nach 10 Sekunden und nach 3 Minuten gemessen. Die Meßwerte wurden als Differenz zwischen den 10 Sekunden- und 3 Minutenwerten berechnet.

Die Referenzkurve für die Messung des Standards wurde auf halblogarithmischem Papier mit Konzentrationen auf der Abszisse und den Extinktionswerten auf der Ordinate gezeichnet und daran wurden die Meßwerte für die Patientenseren ausgewertet.

## Ansprüche

1. Dispersionspolymer aus einer Verbindung oder mehreren Verbindungen der Formel I

$$CH_2 = CR_1\text{-}CO\text{-}NH\text{-}(CH_2)_n\text{-}CH(OR_2)OR_3 \quad I$$

worin
n = 1 - 6 und
$R_1$ = H oder $CH_3$ sind und
$R_2$ und $R_3$ gleich oder unterschiedlich sind und
$-(CH_2)_m\text{-}CH_3$ mit m 0 - 7 oder
$-C(X)(Y)Z$ mit X, Y und Z = $(CH_2)_pCH_3$ und
p = 1 - 3, wobei X, Y, und Z gleich oder unterschiedlich sein können,
bedeuten, und gegebenenfalls einem weiteren Vinylderivat oder weiteren Vinylderivaten in einem wässrigen Medium.

2. Polymer nach Anspruch 1, worin die Verbindung der Formel I Acryl- oder Methacrylamidoalkanal-dialkylacetal mit $C_2$- bis $C_8$-Alkyl ist.

3. Polymer nach Anspruch 1, worin die Verbindung der Formel I Acryl- oder Methacrylamidoacet aldehyd-di-n-pentylacetal ist.

4. Polymer nach Anspruch 1, dadurch gekennzeichnet, daß ein weiteres Vinylpolymeres Styrol, Vinylnaphthalin, Vinyltoluol, Methacrylsäure, Acrylsäure oder Crotonsäure ist.

5. Polymer nach Anspruch 1, dadurch gekennzeichnet, daß ein weiteres Vinylpolymeres ein wasserlösliches Monomeres aus der Gruppe der hydroxy-substituierten Acryl- oder Methacrylsäureester oder Amide oder von wasserlöslichen Monomeren mit Betainstruktur ist.

6. Verfahren zur Herstellung eines Polymeren, dadurch gekennzeichnet, daß eine Verbindung oder mehrere Verbindungen der Formel I und gegebenenfalls ein weiteres Vinylderivat in einem wässrigen Medium in Gegenwart eines Emulgators und eines radikalbildenden Initiators polymerisiert werden.

7. Verwendung eines Polymeren oder Copolymeren nach Anspruch 1 zur Herstellung eines Konjugats mit einer biologisch aktiven Substanz.

8. Verwendung eines Polymeren oder Copolymeren nach Anspruch 1 zur Herstellung eines Konjugats mit einem Immunglobulin.

9. Verwendung eines Polymeren oder Copolymeren nach Anspruch 1 zur Herstellung eines Konjugats mit Antikörpern gegen C-reaktives Protein (CRP), gegen alphaFetoprotein (AFP) oder gegen Immunglobulin E (IgE).

Patentansprüche für den folgenden Vertragsstaat: ES

1. Verfahren zur Herstellung eines Polymeren, dadurch gekennzeichnet, daß eine Verbindung oder mehrere Verbindungen der Formel I

$$CH_2 = CR_1\text{-}CO\text{-}NH\text{-}(CH_2)_n\text{-}CH(OR_2)OR_3 \qquad I$$

worin
n = 1 - 6 und
$R_1$ = H oder $CH_3$ sind und
$R_2$ und $R_3$ gleich oder unterschiedlich sind und
-$(CH_2)_m$-$CH_3$ mit m 0 - 7 oder
-C(X)(Y)Z mit X, Y und Z = $(CH_2)_p CH_3$ und
p = 1 - 3, wobei X, Y, und Z gleich oder unterschiedlich sein können,
bedeuten, und gegebenenfalls ein weiteres Vinylderivat oder weitere Vinylderivate in einem wässrigen Medium in Gegenwart eines Emulgators und einem radikalbildenden Initiator polymerisiert werden.

2. Verfahren nach Anspruch I, worin die Verbindung der Formel I Acryl- oder Methacrylamidoalkylaldehyd-dialkylacetal mit $C_2$- bis $C_8$-Alkyl ist.

3. Verfahren nach Anspruch 1, worin die Verbindung der Formel I Acryl- oder Methacrylamidoalkylaldehyd-di-n-penylacetal ist.

4. Verfahren nach Anspruch I, dadurch gekennzeichnet, daß ein weiteres Vinylpolymeres Styrol, Vinylnaphthalin, Vinyltoluol, Methacrylsäure, Acrylsäure oder Crotonsäure ist.

5. Verfahren nach Anspruch 1, dadurch gekennzeichnet, daß ein weiteres Vinylpolymeres ein wasserlösliches Monomeres aus der Gruppe der hydroxy-substituierten Acryl- oder Methacrylsäureester oder Amide oder von wasserlöslichen Monomeren mit Betainstruktur ist.

FIG. 1

FIG. 2

FIG. 3